# FASCICULE DE BREVET EUROPEEN

(11) **EP 0 356 317 B1**
(45) Date de publication et mention de la délivrance du brevet: **27.10.1993**
(21) Numéro de dépôt: 89402292.0
(22) Date de dépôt: 16.08.1989
(51) Int. Cl.: C07C 67/56, C07C 67/58, C07C 69/24, C07C 69/52

(54) **Procédé de purification d'un ester**
Verfahren zur Reinigung eines Esters
Process for the purification of an ester

(30) Priorité: 22.08.1988 FR 8811151
(43) Date de publication de la demande: 28.02.1990
(73) Titulaire: INSTITUT FRANCAIS DU PETROLE, 92506 Rueil-Malmaison Cédex (FR)
(72) Inventeur: Stern, Robert, F-75017 Paris (FR); Hillion, Gérard, F-95220 Herblay (FR)

(56) Documents cités:
- US-A- 4 695 411

## Description

L'invention concerne un nouveau procédé de purification d'esters.

La purification des esters a pour but d'éliminer les produits pouvant avoir des effets négatifs dans certaines utilisations des esters, comme, par exemple, la substitution du gazole dans les moteurs Diesels; ainsi, la présence de sels alcalins, qui sont des catalyseurs utilisés dans la fabrication d'esters, peut favoriser la formation de dépôts sur les injecteurs. Dans d'autres usages des esters, la présence de ces sels peut notamment colorer une résine alkyde, empoisonner un catalyseur d'hydrogénation (quand l'ester est soumis à une hydrogénation) : par exemple, le chromite de cuivre, qui est un catalyseur d'hydrogénation et d'hydrogénolyse des esters, est empoisonné par des traces de métaux alcalins. Enfin, la présence de traces d'acides minéraux, qui sont aussi des catalyseurs utilisés dans la fabrication des esters, peut provoquer des colorations et l'hydrolyse de l'ester et surtout avoir des effets de corrosion.

Il est donc indispensable d'éliminer, de préférence au moins jusqu'au niveau de la partie par million (1ppm) en poids, tout catalyseur introduit dans l'ester par exemple lors de la fabrication de celui-ci.

Dans son brevet US-A-4 695 411, la demanderesse a proposé de traiter, après leur fabrication, des esters éthyliques ou des mélanges d'esters méthylique et éthylique ou butylique et éthylique par des lavages successifs à l'eau (suivis d'un ajout d'alcool) ou par passage sur des résines échangeuses d'ions cationiques ou par passage sur terre activée.

La méthode de purification par passage sur résines échangeuses d'ions cationiques est une méthode élégante, mais appliquée directement après une fabrication d'esters dont la dernière étape a vu l'introduction de bases, elle exige des consommations très élevées et des régénérations très fréquentes de résines.

En effet, la régénération d'une résine cationique utilisée dans un effluent non aqueux est plus complexe qu'une régénération habituelle. Il faut, avant de passer l'eau et l'acide minéral servant d'agent régénératif déplacer d'abord l'ester présent dans la résine et aussi la glycérine souvent présente lorsque l'ester a été fabriqué notamment à partir d'une huile végétale ou animale; cette glycérine se loge généralement dans les pores des résines échangeuses d'ions. Ce déplacement se fait de préférence avec l'alcool présent et réactif qui est, dans le cas des esters méthylique et éthylique, l'alcool méthylique ou éthylique. De même, après la régénération et le lavage de la résine à l'eau jusqu'à la quasi-neutralité, il faut déplacer à nouveau l'eau par l'alcool; la consommation en alcool n'est donc pas négligeable et correspond à plusieurs fois le volume de la résine.

Il faut donc chercher à régénérer le moins souvent possible cette résine. Le raisonnement précédent est également valable pour les résines échangeuses d'ions anioniques, qui sont régénérés par des bases.

On a maintenant trouvé, de manière surprenante, qu'il est aisé d'augmenter la durée de vie de la résine avant toute régénération, par un procédé simple de purification d'esters qui notamment ne crée pas de source de polluants et peut améliorer sensiblement le rendement en glycérine (produit à grande valeur ajoutée).

Le procédé de purification d'un ester selon l'invention consiste à combiner lavage(s) à l'eau et passage sur résine(s) échangeuse(s) d'ions.

Le procédé de purification selon l'invention s'applique de manière avantageuse aux esters d'acides gras (par exemple de C₆ à C₃₀) et de monoalcools (par exemple le méthanol, l'éthanol, le propanol, le butanol) ou de polyalcools partiellement ou totalement substitués (par exemple les glycols (notamment l'éthylèneglycol, le propylèneglycol, le polyéthylèneglycol), les triols (notamment la glycérine (glycérol), la pentaglycérine (triméthyloléthane), l'hexaglycérine (triméthylolpropane)), les tétrols (notamment l'érythritol), le glucose, la saccharose, aux esters d'acides aromatiques (par exemple l'acide phtalique, l'acide téréphtalique, l'acide benzoïque) et de monoalcools ou de polyalcools partiellement ou totalement substitués, aux esters de polyacides (par exemple les dimères d'acides gras, les trimères d'acides gras, l'acide adipique, l'acide subérique, l'acide pimélique, l'acide dodécanedioïque) et de monoalcools ou de polyalcools partiellement ou totalement substitués.

Le procédé selon l'invention permet notamment de purifier tous les esters de corps gras obtenus à partir de graisses ou d'huiles, telles que par exemple l'huile de colza, l'huile de lin, l'huile de ricin, l'huile de coton, l'huile de palme, l'huile d'arachide, l'huile de tournesol, l'huile de soja, l'huile de maïs, l'huile d'amande, l'huile de coprah, l'huile de carthame, l'huile de pulghere, l'huile d'olive, l'huile de kapok, l'huile de datte amère, l'huile de papayer, l'huile de coloquinte, l'huile de croton, l'huile de souchet, l'huile d'épurge, l'huile de chanvre, l'huile d'hêtre, l'huile de ketmie, l'huile de cameline, l'huile de niger, l'huile d'hévéa, l'huile de noyer, l'huile de noix fièvre, l'huile de sorgho, l'huile de pépin de raisin, l'huile de tabac, l'huile d'afzellie, l'huile de chou navet, l'huile de moutarde brune, l'huile d'aleurite, l'huile de sapin, l'huile de périlla, l'huile de sésame, le talloil, le beurre, le saindoux, les suifs, les huiles de poisson, l'huile de blanc de baleine, les graisses d'animaux terrestres et maritimes, les graisses végétales.

Les esters à purifier suivant le procédé de l'invention ne doivent généralement pas donner d'émulsion et sont insolubles ou très peu solubles dans l'eau.

L'invention propose donc un procédé de purification d'un ester contenant au moins une partie du catalyseur basique employé lors de sa fabrication, ledit ester étant utilisable comme substitut de carburant Diesel, ledit procédé étant caractérisé en ce que, pour obtenir une teneur en catalyseur inférieure à 1 ppm en poids, on met en oeuvre
a) une étape de lavage dudit ester au moyen d'une quantité d'eau représentant en poids de 0,5 à 10% du poids dudit ester ;
b) une étape de récupération, après décantation, d'une phase renfermant ledit ester ;
c) éventuellement une nouvelle étape de lavage telle que (a) et une nouvelle étape de décantation telle que (b) ; et
d) une étape de passage de ladite phase renfermant l'ester provenant de l'étape (b) ou de l'étape (c) sur une résine échangeuse d'ions cationique.

Ainsi, dans le procédé selon l'invention, on peut soit effectuer un labage à l'eau suivi d'une décantation, puis un passage sur au moins une résine échangeuse d'ions, soit effectuer deux lavages à l'eau, avec une décantation entre ces deux lavages et une décantation après le second lavage, puis un passage sur au moins une résine échangeuse d'ions.

Dans le procédé selon l'invention, on peut aussi d'une part, employer de l'eau acide (dont le pH est de préférence compris entre 1 et 5) au moins dans l'étape c) (c'est à dire faire un premier lavage (étape a)) à l'eau acide ou non, et, après décantation, un second lavage (étape c)) à l'eau acide), et d'autre part, dans l'étape d), faire passer la phase renfermant l'ester et provenant de l'étape c) successivement sur deux résines échangeuses d'ions, l'une, de préférence la première, étant une résine échangeuses d'ions cationiques, l'autre, de préférence la seconde, étant une résine échangeuses d'ions anioniques.

Dans le procédé selon l'invention, on peut encore, d'une part, employer de l'eau acide (dont le pH est de préférence compris entre 1 et 5) dans l'étape a), et d'autre part, dans l'étape d), faire passer la phase renfermant l'ester et provenant de l'étape b)(il n'y a donc pas de second lavage, donc pas d'étape c)) successivement sur deux résines échangeuses d'ions, l'une, de préférence la première, étant une résine échangeuses d'ions cationiquees, l'autre, de préference la seconde, étant une résine échangeuses d'ions anioniques.

L'eau acide contient au moins un acide minéral (habituellement fort), par exemple l'acide sulfurique, l'acide phosphorique, l'acide chlorhydrique, ou/et au moins un acide organique dont les sels organiques sont solubles dans l'eau, par exemple l'acide oxalique, l'acide acétique, l'acide formique.

Lors de chaque lavage (étape a) ou étape c)),la quantité d'eau (ou d'eau acide) représente de préférence 2 à 4% en poids du poids de l'ester à purifier.

Les résines cationiques employées dans le procédé selon l'invention sont de préférence fortement acides et macro-réticulées: on peut utiliser par exemple la résine Amberlyst-15, commercialisée sous cette marque par la société Rohm et Haas.

Les résines anioniques employées dans le procédé selon l'invention sont de préférence faiblement ou moyennement basiques et macro-réticulées: on peut utiliser par exemple la résine IRA-93 SP, commercialisée sous cette marque par la société Rohm et Haas.
A l'issue du procédé de purification selon l'invention, l'ester renferme, de préférence, moins d'1ppm, et avantageusement, moins de 0,5ppm en poids de catalyseur.

Le catalyseur contenu dans l'ester à purifier est généralement le catalyseur utilisé lors de la fabrication de cet ester (qui est habituellement une transestérification ou une estérification) et dont une certaine quantité est encore présente dans l'ester (avec éventuellement de l'alcool) à la fin de ladite fabrication.

Comme catalyseurs basiques utilisés dans la fabrication des esters, on peut envisager notamment des bases comme la soude ou la potasse, des sels alcalins, les alcoolates de sodium ou de potassium, des carbonates alcalins.

Si, lors de la transestérification, on a procédé à une double catalyse acide puis basique, le procédé de purification de l'ester fabriqué est le même que le procédé de purification d'un ester obtenu par une catalyse basique uniquement, car l'ester ne contient alors généralement plus de traces de catalyseur acide.

On rappelle à ce propos que les catalyseurs acides utilisés dans la fabrication des esters sont notamment des acides minéraux tels que, par exemple, l'acide sulfurique, l'acide chlorhydrique, des acides organiques tels que, par exemple, les acides sulfoniques (en particulier l'acide toluène, xylène, benzène, naphtalènesulfonique, les acides dodécyl et didodécylbenzène sulfoniques, les acides phénolsulfoniques...).

Dans le procédé selon l'invention, lors de chaque lavage à l'eau (acide ou non), l'ester et l'eau ainsi ajoutée sont en général soumis à une agitation pendant l'addition de cette eau puis, après cette addition, pendant quelques minutes (généralement 2 à 20 minutes), à une température habituellement comprise entre environ 30 et 70°C, de préférence entre environ 40 et 60°C.

La décantation intervient après l'arrêt de l'agitation; ce peut être notamment une décantation statique (on obtient une superposition de deux phases par exemple) ou une décantation dynamique telle qu'une centrifugation.

Le lavage à l'eau acide ou non (étape a) du procédé selon l'invention puis la décantation permettent d'obtenir deux phases :
- une phase aqueuse contenant la majeure partie de l'eau de lavage, la majeure partie du catalyseur (en général 70 à 90% en poids du poids du catalyseur présent dans l `ester avant l'étape a)), et, généralement, de l'alcool et/ou de la glycérine, et
- une phase contenant l'ester (c'est la phase supérieure dans le cas d'une décantation statique par exemple) et le reste du catalyseur (et éventuellement une petite quantité d'eau, d'alcool et/ou de glycérine).

C'est cette seconde phase qui sera soumise à l'étape c) du procédé selon l'invention (si on estime notamment que la quantité de catalyseur encore présent dans l'ester est trop importante) ou directement à l'étape d) du procédé selon l'invention.

L'étape d) permet d'éliminer au moins quasi-totalement le reste de catalyseur encore présent dans l'ester après l'étape b) ou l'étape c) : ainsi, après l'étape d), l'ester renferme, de préférence, moins d'1ppm en poids de catalyseur.

Dans le cas où l'ester à purifier contient également de la glycérine ou/et des traces de monoglycérides (et en général de l'alcool), le procédé de purification selon l'invention permet de récupérer cette glycérine et/ou ces traces de monoglycérides : en effet, cette glycérine et/ou ces traces de monoglycérides se retrouvent au moins en majeure partie, lors de la décantation, dans la phase aqueuse (lors de l'étape b)), car elles sont plus solubles dans un mélange alcool + eau que dans l'ester. Cette glycérine peut être ajoutée à la glycérine généralement recueillie lors de la fabrication de l'ester, ce qui permet par conséquent d'augmenter le rendement en glycérine qui est un produit à haute valeur ajoutée; on évite aussi, grâce au(x) lavage(s) à l'eau, d'avoir une accumulation trop élevée de glycérine sur la résine (dans l'étape d)) et donc des régénérations trop fréquentes de cette résine.

L'alcool généralement présent dans la phase aqueuse peut être récupéré en soumettant ladite phase par exemple à un simple "flash" d'alcool; ceci permet éventuellement d'obtenir une phase aqueuse (constituée presque totalement d'eau) qui est non polluante et qui peut ainsi être déversée sans danger dans la nature.

Le(s) lavage(s) à l'eau acide permet(tent) de réduire très sensiblement la teneur en catalyseur dans l'ester à purifier (avant passage sur résine(s)), par exemple dans certains cas jusqu'à 0,1 à 0,7 mole par tonne de phase ester (phase contenant l'ester) ce qui entrainera une consommation en résine cationique ou anionique de seulement 0,1 à 1 litre par tonne de phase ester.

De plus , le lavage à l'eau permet de réduire considérablement, voire d'éliminer, la quantité d'acides gras éventuellement présents dans l'ester, car ils sont éliminés dans la phase aqueuse.

Après l'étape d), on peut évaporer l'alcool et/ou les traces d'eau éventuellement encore présents dans l'ester ou ajouter de l'alcool (quelques pour cent) à cet ester. La présence d'alcool dans l'ester jusqu'à environ 20% en poids ne perturbe pas en général le fonctionnement d'un moteur, bien que l'indice de cétane soit diminué et le point éclair abaissé.

On peut employer au moins un ester purifié par le procédé selon l'invention dans une composition utilisable comme carburant de substitution de gazole (notamment dans les moteurs Diesel).

Il faut enfin noter que toutes les étapes comme la catalyse (fabrication de l'ester), le(s) lavage(s), la (les) décantation(s), le passage sur résine(s) peuvent être effectuées en continu.

L'invention est illustrée par les exemples suivants non limitatifs.

### EXEMPLE 1

Une tonne d'huile de colza dégommée et légèrement acide est soumise à un traitement de transestérification qui comprend l'introduction en deux temps d'alcool méthylique (256 litres) et de soude (5,5 kilogrammes). Après décantation, l'ester obtenu a une pureté de 97,5% par rapport aux composés huileux de la phase ester. La pureté est mesurée par indice de réfraction de l'ester lavé et séché par comparaison avec un ester pur et l'huile. L'ester contient en outre 3 à 5% en poids d'alcool, des traces de monoglycérides et diglycérides et surtout des traces de sodium en une quantité représentant 20,5 millimoles d'ions sodium par kilogramme de phase ester.

En lavant l'ester avec environ 20 litres d'eau (étape a) du procédé selon l'invention) et en agitant pendant 10 minutes à 50°C, on obtient, après décantation et soutirage de la phase aqueuse, une phase ester qui titre 7,7 millimoles d'ions sodium par kilogramme de phase ester (étape b) du procédé selon l'invention).

On fait ensuite passer cette phase ester (étape d) du procédé selon l'invention) à travers une résine échangeuse d'ions cationiques Amberlyst-15 (60 litres environ); on obtient un ester contenant moins de 0,5ppm en poids d'ions sodium (environ moins de 0,01 millimole d'ions sodium/kg d'ester).

De même, la teneur pondérale en glycérine de l'ester, qui atteignait 940ppm dans l'ester après la décantation qui suivait la fabrication dudit ester, se réduit à 380ppm après le lavage à l'eau et à 2ppm après le passage sur résine Amberlyst-15 (378ppm de glycérine s'étant donc fixés sur cette résine).

D'une part, on a donc réduit très sensiblement la quantité d'ions sodium dans l'ester, notamment avant le passage sur la résine Amberlyst-15, ce qui a permis de consommer environ 7,5 litres de résine par tonne de phase ester au lieu des 20,5 litres de résine que l'on aurait consommés par tonne de phase ester si on n'avait pas effectué le lavage à l'eau (1 litre de résine fixant environ 1 mole d'ions sodium); d'autre part, grâce au lavage à l'eau, on a réduit d'environ 60% la quantité de glycérine fixée sur la résine (378ppm contre 938ppm environ qui se seraient fixés si le lavage à l'eau n'avait pas été opéré). Enfin les monoglycérides ont pratiquement été toutes éliminées par le lavage à l'eau.

### EXEMPLE 2

On fabrique, de manière analogue à l'exemple 1, un ester à partir d'une tonne d'huile de colza dégommée et légèrement acide.

On lave ensuite l'ester avec environ 20 litres d'eau (étape a) du procédé selon l'invention) et on agite pendant dix minutes à 50°C; on obtient, après décantation et soutirage de la phase aqueuse, une phase ester qui titre 7,7 millimoles d'ions sodium par kilogramme de phase ester (étape b) du procédé selon l'invention).

Puis on lave ladite phase ester avec environ 20 litres d'eau acide (eau contenant environ 7,5 moles équivalent d'acide sulfurique, ce qui correspond à l'alcalinité de l'ester), on agite pendant 10 minutes à 50°C et on obtient, après décantation et soutirage de la phase aqueuse, une phase ester qui titre 0,3 millimole d'ions sodium par kilogramme de phase ester (étape c) du procédé selon l'invention).

On fait ensuite passer cette phase ester (étape d) du procédé selon l'invention) à travers une résine échangeuse d'ions cationiques Amberlyst-15 (60 litres environ) puis à travers une résine échange d'ions anioniques IRA-93 SP (60 litres environ); on obtient un ester contenant moins de 0,5ppm en poids d'ions sodium (environ moins de 0,01 millimole d'ions sodium/kg d'ester).

On a donc réduit très sensiblement la quantité d'ions sodium dans l'ester, notamment avant le passage sur les deux résines, ce qui a permis de ne consommer au total que 0,5-0,6 litre de résines par tonne de phase ester.

### EXEMPLE 3

On veut purifier un ester éthylique d'huile de colza obtenu par une catalyse acide suivie de deux catalyses basiques (à l'aide de soude) et contenant 4,5 millimoles d'ions sodium par kilogramme d'ester.
. Si l'on passe directement (sans aucun lavage) l'ester à travers une résine échangeuse d'ions cationiques Amberlyst-15, on consommera environ 4,5 litres de résine par tonne d'ester et, avec une colonne de résine Amberlyst-15 de 60 litres environ, on n'obtiendra finalement qu'environ 13 tonnes d'ester avant régénération de ladite résine.
. Au contraire, si on lave l'ester (étape a) du procédé selon l'invention) avec environ 20 litres d'eau acide (eau contenant environ 7,5 moles équivalent d'acide sulfurique) et on agite pendant 10 minutes à 50°C, on obtient, après décantation et soutirage de la phase aqueuse, une phase ester qui titre 0,15 millimole d'ions sodium par kilogramme de phase ester (étape b) du procédé selon l'invention).
On fait ensuite passer cette phase ester (étape d) du procédé selon l'invention) à travers une résine échangeuse d'ions cationiques Amberlyst-15 (60 litres environ), puis à travers une résine échangeuse d'ions anioniques IRA-93 SP (60 litres environ); on obtient un ester contenant moins de 0,5 ppm en poids d'ions sodium (environ moins de 0,01 millimole d'ions sodium/kg d'ester). La consommation totale en résines est inférieure à 0,4 litre par tonne de phase ester. Avec une colonne de résine Amberlyst-15 et une colonne de résine IRA-93 SP de 60 litres chacune environ, on obtient donc environ 300 tonnes d'ester avant régénération de ladite résine (contre 13 tonnes si le lavage à l'eau acide n'est pas effectué).

### EXEMPLE 4

On veut purifier un ester méthylique d'huile de colza obtenu par catalyse basique (à l'aide de soude) et contenant 11,3 millimoles d'ions sodium par kilogramme d'ester.

On lave 3 kilogrammes de cet ester avec 55 grammmes d'eau (étape a) du procédé selon l'invention) dans une centrifugeuse de laboratoire (à 50°C environ). Après centrifugation et soutirage de la phase aqueuse, on obtient une phase ester qui titre 0,127 millimole d'ions sodium par kilogramme de phase ester (étape b) du procédé selon l'invention). Le traitement à l'eau est ici assez efficace pour qu'il ne soit pas nécessaire d'effectuer un deuxième lavage à l'eau (ou à l'eau acide).

On fait ensuite passer cette phase ester (étape d) du procédé selon l'invention) à travers une résine échangeuse d'ions cationiques Amberlyst-15 (60 litres environ); on obtient un ester contenant moins de 0,5ppm en poids d'ions sodium (environ moins de 0,01 millimole d'ions sodium/kg d'ester). La consommation en résine a été d'environ 0,13 litre par tonne de phase ester, alors qu'elle aurait été d'environ 12 litres par tonne d'ester si on n'avait pas effectué le lavage à l'eau. De plus, on a récupéré la quasi totalité de la glycérine dans la phase aqueuse.

### EXEMPLE 5

On veut purifier un ester méthylique conjugué d'huile de colza obtenu en présence d'alcoolate de potassium et contenant 150 millimoles d'ions potassium par kilogramme d'ester.

On lave ledit ester (étape a) du procédé selon l'invention) avec environ 20 litres d'eau acide (eau contenant 150 moles équivalent d'acide sulfurique, ce qui correspond à l'alcalinité de l'ester), on agite pendant 10 minutes à 50°C et on obtient, après décantation et soutirage de la phase aqueuse, une phase ester qui titre 1,1 millimole de potassium par kilogramme de phase ester (étape b) du procédé selon l'invention).

On fait ensuite passer cette phase ester (étape d) du procédé selon l'invention) à travers une résine échangeuse d'ions cationiques Amberlyst-15 (60 litres environ) puis à travers une résine échangeuse d'ions anioniques IRA-93 SP (60 litres environ); on obtient un ester contenant moins de 0,5ppm en poids d'ions potassium.

On a donc, d'une part, réduit très sensiblement la quantité d'ions potassium dans l'ester, notamment avant le passage sur les deux résines, ce qui a permis de ne consommer au total que 1-2 litres de résines par tonne de phase ester, alors que l'on aurait consommé 150 litres de résine par tonne d'ester si on n'avait pas effectué de lavage.

## Revendications

1. Procédé de purification d'un ester contenant au moins une partie du catalyseur basique employé lors dc sa fabrication, ledit ester étant utilisable comme substitut de carburant Diesel, ledit procédé étant caractérisé en ce que, pour obtenir une teneur en catalyseur inférieure à 1 ppm en poids, on met en oeuvre
a) une étape de lavage dudit ester au moyen d'une quantité d'eau représentant en poids de 0,5 à 10 % du poids dudit ester ;
b) une étape de récupération, après décantation, d'une phase renfermant ledit ester ;
c) éventuellement une nouvelle étape de lavage telle que (a) et une nouvelle étape de décantation telle que (b) ; et
d) une étape de passage de ladite phase renfermant l'ester provenant de l'étape (b) ou de l'étape (c) sur une résine échangeuse d'ions cationique.

2. Procédé selon la revendication 1, caractérisé en ce que l'eau employée, au moins dans l'étape c), est acide et en ce que, dans l'étape d), on fait passer la phase renfermant l'ester et provenant de l'étape c) sur une résine échangeuse d'ions cationique, puis sur une résine échangeuse d'ions anionique.

3. Procédé selon la revendication 1, caractérisé en ce que l'eau employée dans l'étape a) est acide et en ce que dans l'étape d), on fait passer la phase renfermant l'ester et provenant de l'étape b) sur une résine échangeuse d'ions cationique, puis sur une résine échangeuse d'ions anionique.

4. Procédé selon l'une des revendications 1 à 3, caractérisé en ce que la quantité d'eau employée lors de chaque lavage à l'eau représente 2 à 4 % en poids du poids dudit ester.

5. Procédé selon l'une des revendications 2 à 4, caractérisé en ce que l'eau acide employée a un pH compris entre 1 et 5.

6. Procédé selon l'une des revendications 1 à 5, caractérisé en ce que ledit ester résulte de la transestérification par catalyse basique d'au moins un ester d'acide gras de C₆ à c₃₀ et de glycérol, par un monoalcool.

7. Procédé selon l'une des revendications 1 à 5, caractérisé en ce que ledit ester résulte de la transestérification par catalyse acide puis basique d'au moins un ester d'acide gras de C₆ à C₃₀ et de glycérol, par un monoalcool.

8. Procédé selon l'une des revendications 6 et 7, caractérisé en ce que ledit ester d'acide gras de C₆ à C₃₀ et de glycérol est une graisse ou une huile d'origine végétale ou animale.

9. Procédé selon l'une des revendications 1 à 8, caractérise en ce que ledit catalyseur basique est choisi parmi la soude, la potasse, des sels alcalins, les alcoolates de sodium ou de potassium, et les carbonates alcalins.

10. Procédé selon l'une des revendications 1 à 9, dans lequel la température du mélange ester + eau est maintenue dans l'étape a) entre environ 40 et 60° C pendant 2 à 20 minutes.

11. Procédé selon l'une des revendications 1 à 10, dans lequel, après l'étape d), on évapore l'alcool et les traces d'eau présents dans l'ester.

12. Procédé selon l'une des revendications 1 à 10. dans lequel, après l'étape d), on ajoute de l'alcool à l'ester.

13. Procédé selon l'une des revendications 1 à 12, à l'issue duquel ledit ester renferme moins d'1 ppm en poids de catalyseur basique.

## Claims

1. A process for purifying an ester containing at least a part of the basic catalyst used for its manufacture, said ester being usable as a substitute for Diesel fuel, said process being characterized in that, for obtaining a catalyst content lower than 1 ppm by weight, it involves
a) a step of washing said ester by means of a quantity of water that represents by weight from 0.5 to 10% of the weight of said ester:
b) a step of recovering, after settling, a phase containing said ester:
c) optimally a further step of washing such as step (a) and a further step of settling such as step (b): and
d) a step of circulating said phase containing the ester issuing from step(b) or step(c) in contact with a cationic ion-exchange resin.

2. A process according to claim 1, characterized in that the water used at least in step (c) is acid, and in that, in step (d) the phase containing the ester and issuing from step (c) is circulated in contact with a cationic exchange resin, then in contact with an anionic ion exchange resin.

3. A process according to claim 1, characterized in that the water used in step (a) is acid, and in that, in step (d) the phase containing the ester, and issuing from step (b) is circulated in contact with a cationic ion-exchange resin, then in contact with an anionic ion-exchange resin.

4. A process according to one of claims 1 to 3 characterized in that the quantity of water used for each water-washing step represents by weight 2 to 4% of the weight of said ester.

5. A process according to one of claims 2 to 4, characterized in that the acid water has a pH comprised between 1 and 5.

6. A process according to one of claims 1 to 5, characterized in that said ester results from the transesterification by basic catalysis of at least one ester of C₆-C₃₀ fatty acid and glycerol with a monoalcohol.

7. A process according to one of claims 1 to 5, characterized in that said ester results from the transesterification by acid catalysis then basic catalysis of at least one ester of C₆-C₃₀ fatty acid and glycerol with a monoalcohol.

8. A process according to one of claims 6 and 7, characterized in that said ester of C₆-C₃₀ fatty acid and glycerol is a grease or an oil of plant or animal origin.

9. A process according to one of claims 1 to 8, characterized in that said basic catalyst is selected for soda, potash, alcaline salts, sodium or potassium alcoholates and alcaline carbonates.

10. A process according to one of claims 1 to 9, wherein, in step (c), the temperature of the mixture ester + water is maintained between about 40 and 60°C for 2-20 minutes.

11. A process according to one of claims 1 to 10, wherein, after step (d), the alcohol and the water traces present in the ester are evaporated.

12. A process according to one of claims 1 to 10, wherein, after step (d), the alcohol is being added to the ester.

13. A process according to one of claims 1 to 12, after which said ester contains less than 1 ppm by weight of basic catalyst.

## Patentansprüche

1. Verfahren zur Reinigung eines Esters, der mindestens einen Teil des bei seiner Herstellung verwendeten basischen Katalysators enthält und der als Ersatz für Diesel-Kraftstoff verwendbar ist, dadurch gekennzeichnet, daß man zur Erzielung eines Katalysator-Gehaltes von weniger als 1 Gew.-ppm durchführt:
a) eine Waschstufe, in welcher der genannte Ester mit einer Menge Wasser, die 0,5 bis 10 % des Gewichts des genannten Esters entspricht, gewaschen wird;
b) eine Abtrennungsstufe, in der eine den genannten Ester enthaltende Phase nach der Dekantation abgetrennt (gewonnen) wird;
c) gegebenenfalls eine erneute Waschstufe wie (a) und eine erneute Dekantierungsstufe wie (b); und
d) eine Durchlaufstufe, in der man die genannte Phase, die den aus der Stufe (b) oder aus der Stufe (c) stammenden Ester enthält, über ein Kationenaustauscherharz laufen läßt.

2. Verfahren nach Anspruch 1, dadurch gekennzeichnet, daß das mindestens in der Stufe (c) verwendete Wasser sauer ist und man in der Stufe (d) die den Ester enthaltende Phase, die aus der Stufe (c) stammt, zuerst über ein Kationenaustauscherharz und dann über ein Anionenaustauscherharz laufen läßt.

3. Verfahren nach Anspruch 1, dadurch gekennzeichnet, daß das in der Stufe (a) verwendete Wasser sauer ist und man in der Stufe (d) die den Ester enthaltende Phase, die aus der Stufe (b) stammt, zuerst über ein Kationenaustauscherharz und dann über ein Anionenaustauscherharz laufen läßt.

4. Verfahren nach einem der Ansprüche 1 bis 3, dadurch gekennzeichnet, daß die in der Waschstufe verwendete Wassermenge 2 bis 4 % des Gewichts des genannten Esters entspricht.

5. Verfahren nach einem der Ansprüche 2 bis 4, dadurch gekennzeichnet, daß das verwendete Wasser einen pH-Wert zwischen 1 und 5 hat.

6. Verfahren nach einem der Ansprüche 1 bis 5, dadurch gekennzeichnet, daß der genannte Ester resultiert aus der Umesterung mindestens eines C₆-C₃₀-Fettsäure-Glycerin-Esters durch basische Katalyse mit einem Monoalkohol.

7. Verfahren nach einem der Ansprüche 1 bis 5, dadurch gekennzeichnet, daß der genannte Ester resultiert aus der Umesterung mindestens eines C₆-C₃₀-Fettsäure-Glycerin-Esters durch saure Katalyse und dann durch basische Katalyse mit einem Monoalkohol.

8. Verfahren nach einem der Ansprüche 6 und 7, dadurch gekennzeichnet, daß der genannte C₆-C₃₀-Fettsäure-Glycerin-Ester ein Fett oder ein Öl pflanzlichen oder tierischen Ursprungs ist.

9. Verfahren nach einem der Ansprüche 1 bis 8, dadurch gekennzeichnet, daß der genannte basische Katalysator ausgewählt wird aus Natriumhydroxid, Kaliumhydroxid, Alkalisalzen, Natrium- oder Kaliumalkoholaten und Alkalicarbonaten.

10. Verfahren nach einem der Ansprüche 1 bis 9, in dem die Temperatur des Ester/Wasser-Gemisches in der Stufe (a) 2 bis 20 min lang zwischen etwa 40 und 60°C gehalten wird.

11. Verfahren nach einem der Ansprüche 1 bis 10, in dem nach der Stufe (d) der Alkohol und die Spuren Wasser, die in dem Ester vorhanden sind, verdampft werden.

12. Verfahren nach einem der Ansprüche 1 bis 10, in dem nach der Stufe (d) der Alkohol dem Ester zugesetzt wird.

13. Verfahren nach einem der Ansprüche 1 bis 12, wobei am Ende desselben der Ester weniger als 1 Gew.-ppm basischen Katalysator enthält.
